# EUROPÄISCHE PATENTSCHRIFT

(11) **EP 0 793 642 B1**
(45) Veröffentlichungstag und Bekanntmachung des Hinweises auf die Patenterteilung: **26.01.2000**
(21) Anmeldenummer: 95939274.7
(22) Anmeldetag: 14.11.1995
(51) Int. Cl.: C07C 253/14, C07C 255/40

(54) **VERFAHREN ZUR HERSTELLUNG VON HALOGENMETHYL-BENZOYLCYANIDEN**
PROCESS FOR PREPARING HALOGENATED METHYL BENZOYL CYANIDES
PROCEDE DE PREPARATION DE CYANURES DE METHYLBENZOYLE HALOGENES

(30) Priorität: 24.11.1994 DE 4441824
(43) Veröffentlichungstag der Anmeldung: 10.09.1997
(73) Patentinhaber: BASF AKTIENGESELLSCHAFT, 67056 Ludwigshafen (DE)
(72) Erfinder: ISAK, Heinz, D-67459 Böhl-Iggelheim (DE); KEIL, Michael, D-67251 Freinsheim (DE); WOLF, Bernd, D-67136 Fussgönheim (DE); WINGERT, Horst, D-68159 Mannheim (DE); WETTLING, Thomas, D-67117 Limburgerhof (DE)
(86) Internationale Anmeldenummer: EP9504463
(87) Internationale Veröffentlichungsnummer: WO9616023

(56) Entgegenhaltungen:
- EP-A- 0 079 004
- EP-A- 0 493 711
- DE-C- 4 311 722

## Beschreibung

Die vorliegende Erfindung betrifft ein verbessertes Verfahren zur Herstellung von Halogenmethyl-benzoylcyaniden der allgemeinen Formel I

PH-CO-CN (I)

wobei PH einen Phenylrest bedeutet, der durch Chlormethyl oder Brommethyl substituiert ist und der gewünschtenfalls noch 1 bis 4 weitere, für die Umsetzung inerte Reste tragen kann, aus Halogenmethylbenzoyl-chloriden der allgemeinen Formel II

PH-CO-Cl (II).

EP-A 79004 beschreibt die Umsetzung von u.a. Benzoesäureanhydriden mit Trimethylsilylcyanid zu Benzoylcyaniden gegebenenfalls in Gegenwart eines Katalysators und gegebenenfalls in Gegenwart eines Verdünnungsmittels. Als Katalysatoren kommen neben Basen auch Lewis-Säuren in Frage.

Aus der DE-C 43 11 722 ist bereits bekannt, daß die 2-Halogenmethyl-benzoylchloride II durch Umsetzung mit Alkalimetall- oder Übergangsmetallcyaniden in die entsprechenden 2-Halogenmethyl-benzoylcyanide I übergeführt werden können, und daß der Verfahrensverlauf durch Zugabe eines Katalysators und eines Verdünnungsmittels günstig beeinflußt werden kann. Als geeignete Katalysatoren werden die Halogenide, Cyanide, Hydroxide, Hydrogensulfate, C₁-C₄-Alkylsulfate und Tetrafluoroborate von quartären Stickstoff-Verbindungen sowie Aryl- und Alkyl-Phosphoniumhalogenide genannt. Die in den Beispielen angegebenen Umsetzungen lassen jedoch erkennen, daß die erzielbaren Ausbeuten für eine Umsetzung im technischen Maßstab nicht voll befriedigend sind.

Aufgabe der vorliegenden Erfindung war es demnach, diesem Mangel abzuhelfen.

Demgemäß wurde das vorliegende verbesserte Verfahren zur Herstellung der Halogenmethyl-benzoylcyanide I gefunden, welches dadurch gekennzeichnet ist, daß man II mit einer Cyanid-freisetzenden Verbindung in Gegenwart einer Lewissäure umsetzt, gewünschtenfalls in einem inerten organischen Lösungs- oder Verdünnungsmittel, und danach das Verfahrensprodukt isoliert.

Die Halogenmethyl-benzoylchloride II lassen sich nach bekannten Halogenierungs-Methoden aus den entsprechenden Methyl-substituierten Benzol-Derivaten (vgl. z.B. DE-A 28 35 440 ) oder aus den entsprechenden Benzoesäuren (vgl. z.B. DE-A 40 42 282) herstellen.

Das erfindungegemäße Verfahren wird normalerweise bei Atmosphärendruck oder bei leichtem Unterdruck vorgenommen, wobei die Reaktionstemperatur dann allgemein bei (-20) bis 100°C, vorzugsweise 0 bis 80°C, insbesondere 20 bis 80°C, liegt.

Cyanid-freisetzende Verbindungen sind vorzugsweise Cyanwasserstoff, Cyanhydrin, Alkalimetallcyanide wie Natrium- und Kaliumcyanid oder Übergangsmetallcyanide wie Quecksilber(I)cyanid, Silbercyanid und Kupfer(I)cyanid. Besonders zweckmäßig ist Natriumcyanid.

Im allgemeinen werden das Halogenmethyl-benzoylchlorid II und die Cyanid-freisetzende Verbindung in etwa stöchiometrischen Mengen eingesetzt. Bevorzugt ist jedoch ein Überschuß an Cyanid bis zur 2-fachen Menge, insbesondere an 1,05- bis 1,5-facher Menge, bezogen auf die Menge an II.

Bezüglich des Einsatzes von Lewissäuren sei auf Jerry March, Advanced Organic Chemistry, Fourth Edition 1992, Seiten 539-552 sowie der dort zitierten Literatur verwiesen.

Nach den bisherigen Erkenntnissen kommen vor allem Zinntetrachlorid, Aluminiumchlorid, Eisen(II)chlorid, Eisen(III)chlorid, Zinkchlorid, Titantetrachlorid, Bortrifluorid und Antimonpentachlorid als Katalysatoren in Betracht, wobei Zinntetrachlorid und Titantetrachlorid ganz besonders gut geeignet sind.

Der Katalysator wird vorzugsweise in katalytischen Mengen von 0,01- bis 5-mol-%, insbesondere von 0,1- bis 3-mol-%, bezogen auf die Menge an II, eingesetzt.

Sofern das eingesetzte Halogenmethyl-benzoylchlorid II nicht in flüssiger Form vorliegt, ist der Zusatz eines inerten organischen Lösungs- oder Verdünnungsmittels empfehlenswert, wobei insbesondere aprotische dipolare und unpolare Lösungsmittel geeignet sind.

Unter aprotisch-dipolaren Lösungsmitteln sind solche Lösungsmittel zu verstehen, bei denen ein Lösungsmittelmolekül ein ausgeprägtes Dipolmoment besitzt, jedoch keine Wasserstoffatome trägt, welche zur Ausbildung von Wasserstoffbrücken befähigt sind. Die Dielektrizitätskonstante solcher Lösungsmittel ist größer als 15. Bezüglich der Definition für aprotisch-dipolare Lösungsmittel sei auf A. J. Parker, Chem. Rev. 69 (1969), Seiten 1 - 32, insbesondere Seite 2, verwiesen.

Geeignete aprotisch-dipolare Lösungsmittel sind beispielsweise Sulfoxide wie Dimethylsulfoxid, Diäthylsulfoxid, Dimethylsulfon, Diäthylsulfon, Methyläthylsulfon und Tetramethylensulfon; Nitrile wie Acetonitril, Benzonitril, Butyronitril, Isobutyronitril und m-Chlorbenzonitril; N,N-Dialkyl-substituierte Carbonamide wie Dimethylformamid, Tetramethylharnstoff, N,N-Dimethylbenzamid, N,N-Dimethylacetamid, N,N-Dimethylphenylacetamid, N,N-Dimethylcyclohexancarbonsäureamid, N,N-Dimethylpropionsäureamid und homologes Carbonsäurepiperidid, Carbonsäuremorpholid sowie Carbonsäurepyrrolidid, die entsprechenden N,N-Diäthyl-, N,N-Dipropyl-, N,N-Diisopropyl-, N,N-Diisobutyl-, N,N-Dibenzyl-, N,N-Diphenyl-, N-Methyl-N-phenyl-, N-Cyclohexyl-N-methyl-, N-Äthyl-N-tert.-butyl-Verbindungen der vorstehend genannten N,N-Dimethylverbindungen, des weiteren N-Methyl-formanilid, N-Äthylpyrrolidon, N-Butylpyrrolidon, N-Äthyl-piperidon-(t), N-Methylpyrrolidon und Hexamethylphosphorsäuretriamid. Auch Gemische der genannten Solventien kommen in Betracht.

Bevorzugt sind Dimethylacetamid, N-Methylpyrrolidon, Dimethylformamid, Dimethylsulfoxid Tetramethylensulfon, Aceton und Acetonitril.

Geeignete unpolare Lösungsmittel sind vorzugsweise aromatische Kohlenwasserstoffe wie Benzol, Toluol und o-, m-, p-Xylol, chlorierte Kohlenwasserstoffe wie Methylenchlorid und Alkohole wie Methanol und Ethanol. Besonders bevorzugt ist Toluol. Die Verfahensprodukte I können auf übliche Weise, z.B. mittels Destillation, gereinigt werden.

Das erfindungsgemäße Verfahren kann sowohl diskontinuierlich als auch kontinuierlich durchgeführt werden. Bei der kontinuierlichen Arbeitsweise leitet man die Reaktionspartner beispielsweise durch einen Rohrreaktor oder über Rührkesselkaskaden. Lösungsmittel und Katalysator können gewünschtenfalls im Kreis geführt werden.

Nach dem vorliegenden Verfahren sind die Halogenmethyl-benzoylcyanide I auf technisch einfache Weise mit sehr guter Reinheit und in hoher Ausbeute zugänglich. Eine Reinigung der Rohprodukte ist normalerweise entbehrlich.

Die Halogenmethyl-benzoylcyanide I sind wertvolle Zwischenprodukte zur Herstellung verschiedener Pflanzenschutzmittel, beispielsweise den herbiziden 4-Phenylpyrazolen, wie sie in der EP-A 352 543 beschrieben werden.

Die Verfahrensprodukte I können außerdem zur Synthese von Arylglyoxylsäureestern gemäß der DE-A 40 42 271 dienen. Ein aus der dort beschriebenen Pinner-Reaktion erhaltenes Gemisch aus Phenylglyoxylsäureestern und deren Ketalen kann gemäß der Lehre der DE-A 40 42 272 in die E-Oximether von Phenylglyoxylsäureestern der Formel III 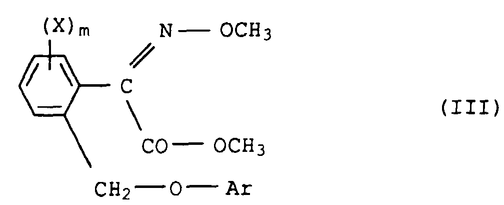 in der Ar für substituiertes oder unsubstituiertes Phenyl steht, übergeführt werden. Verbindungen vom Typ III finden im Pflanzenschutz vorzugsweise als Fungizide, Akarizide oder Insektizide Verwendung (vgl. z.B. EP-A 253 213 und EP-A 254 426).

Im Hinblick auf die aus den Verbindungen I herstellbaren Wirkstoffe III steht Ph vor allem für den Rest 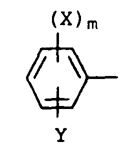 wobei
- X: jeweils Halogen, insbesondere Fluor oder Chlor, C₁-C₄-Alkyl, insbesondere Methyl oder Ethyl, C₁-C₄-Alkoxy, insbesondere Methoxy, Ethoxy oder Isopropoxy, C₁-C₄-Halogenalkyl, insbesondere Trifluormethyl, -C(C₁-C₅-Alkyl)=N-O-(C₁-C₅-alkyl) oder -C(C₁-C₅-Alkyl)=N-O-(C₂-C₅-alkenyl), insbesondere Methylhydroxylimino oder -C(CH₃)=N-OCH₃;
besonders bevorzugt Halogen, C₁-C₄-Alkyl, C₁-C₄-Alkoxy oder C₁-C₄-Halogenalkyl;
- m: 0 bis 4, insbesondere 1, wobei X dann bevorzugt in 3-, 5- oder 6-Position steht,
und
- Y: Chlormethyl oder Brommethyl, insbesondere Chlormethyl
bedeuten, und Y vorzugsweise in 2-Position steht.

Ganz besonders bevorzugt bedeutet Ph Chlormethylphenyl oder Brommethylphenyl.

### Herstellungsbeispiele

### Beispiel 1

### Herstellung von 2-Chlormethyl-benzoylcyanid in Acetonitril

Zu einer Suspension von 196 g (4 mol) Natriumcyanid in 1000 ml Acetonitril wurden 7,8 g (3 mol-%) Zinntetrachlorid gegeben. Anschließend erwärmte man die Mischung auf 60 °C und tropfte dann innerhalb von 30 Minuten 378 g (2 mol) 2-Chlormethyl-benzoylchlorid zu. Nach 6 Stunden Rühren bei ca. 60 °C wurde das Reaktionsgemisch abgekühlt. Danach filtrierte man den ungelösten Katalysator über Kieselgel ab. Alternativ kann der Katalysator auch nach Zugabe von Toluol mit dreimal 200 ml einer verdünnten Mineralsäure aus dem Reaktionsgemisch gewaschen werden.

Die nach Abtrennung des Katalysators verbliebene klare Lösung wurde einer fraktionierten Destillation unterworfen. Ausbeute: 345,8 g (97 %); Sdp_{0,5} = 105°C.

### Beispiel 2

### Herstellung von 2-Chlormethyl-benzoylcyanid in Toluol

Die Herstellung erfolgte analog Beispiel 1, wobei jedoch in 1000 ml Toluol, das bis zu 200 ml Acetonitril enthielt, gearbeitet und nach Zugabe des 2-Chlormethyl-benzoylchlorids 8 Stunden gerührt wurde. Ausbeute: 317,3 g (89 %); Sdp_{0,3} = 100°C.

## Patentansprüche

1. Verfahren zur Herstellung von Halogenmethyl-benzoylcyaniden der allgemeinen Formel I
PH-CO-CN (I)
wobei PH einen Phenylrest bedeutet, der durch Chlormethyl oder Brommethyl substituiert ist und der gewünschtenfalls noch 1 bis 4 weitere, für die Umsetzung inerte Reste tragen kann,
aus Halogenmethylbenzoyl-chloriden der allgemeinen Formel II
PH-CO-Cl (II)
und einer Cyanid-freisetzenden Verbindung dadurch gekennzeichnet, daß man II mit der Cyanid-freisetzenden Verbindung in Gegenwart einer Lewissäure umsetzt, gewünschtenfalls in einem inerten organischen Lösungs- oder Verdünnungsmittel, und danach das Verfahrensprodukt isoliert.

2. Verfahren nach Anspruch 1, dadurch gekennzeichnet, daß die Variable Ph für den Rest 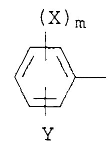 steht, wobei
X jeweils Halogen, C₁-C₄-Alkyl, C₁-C₄-Alkoxy, C₁-C₄-Halogenalkyl, -C(C₁-C₅-Alkyl)=N-O-(C₁-C₅-alkyl) oder -C(C₁-C₅-Alkyl)=N-O-(C₂-C₅-alkenyl),
m 0 bis 4 und
Y Chlormethyl oder Brommethyl
bedeuten.

3. Verfahren nach Anspruch 1, dadurch gekennzeichnet, daß die Cyanid-freisetzende Verbindung ein Alkalimetallcyanid, Übergangsmetallcyanid, Cyanhydrin oder Cyanwasserstoff ist.

## Claims

1. A process for preparing halomethylbenzoyl cyanides of the general formula I
PH-CO-CN (I)
where PH is phenyl which is substituted by chloromethyl or bromomethyl and which can, if required, also carry from 1 to 4 further radicals which are inert in the reaction, from halomethylbenzoyl chlorides of the general formula II
PH-CO-Cl (II)
and a cyanide-donating compound, wherein II is reacted with the cyanide-donating compound in the presence of a Lewis acid, if required in an inert organic solvent or diluent, and then the product is isolated.

2. A process as claimed in claim 1, wherein the variable PH is the radical 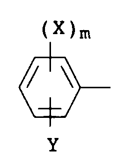 where
X is in each case halogen, C₁-C₄-alkyl, C₁-C₄-alkoxy, C₁-C₄-haloalkyl, -C(C₁-C₅-alkyl)=N-O-(C₁-C₅-alkyl) or -C(C₁-C₅-alkyl)=N-O-(C₂-C₅-alkenyl),
m is 0 to 4 and
Y is chloromethyl or bromomethyl.

3. A process as claimed in claim 1, wherein the cyanide-donating compound is an alkali metal cyanide, transition metal cyanide, cyanohydrin or hydrogen cyanide.

## Revendications

1. Procédé pour la préparation de cyanures d'halogénométhylbenzoyle répondant à la formule générale I
Ph-CO-CN (I)
dans laquelle Ph représente un groupe phényle substitué par un groupe chlorométhyle ou bromométhyle et qui peut encore porter le cas échéant un à quatre autres substituants inertes dans la réaction,
à partir de chlorures d'halogénométhylbenzoyle répondant à la formule générale II
Ph-CO-Cl (II)
et d'un composé libérant un cyanure, caractérisé par le fait que l'on fait réagir II avec le composé libérant un cyanure en présence d'un acide de Lewis, le cas échéant dans un solvant ou diluant organique inerte, puis on isole le produit recherché.

2. Procédé selon la revendication 1, caractérisé par le fait que le symbole Ph représente le groupe 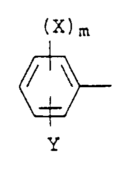 dans lequel chacun des symboles X représente un halogène, un groupe alkyle en C1-C4, alcoxy en C1-C4, halogénoalkyle en C1-C4, -C(alkyle en C1-C5)-N-O-(alkyle en C1-C5) ou -C(alkyle en C1-C5)=N-O-(alcényle en C2-C5),
m est un nombre allant de O à 4 et
Y représente un groupe chlorométhyle ou bromométhyle.

3. Procédé selon la revendication 1, caractérisé par le fait que le composé libérant un cyanure est un cyanure de métal alcalin, un cyanure de métal de transition, une cyanhydrine ou l'acide cyanhydrique.
